# DEMANDE DE BREVET EUROPEEN

(11) **EP 0 704 525 A1**
(43) Date de publication de la demande: **03.04.1996**
(21) Numéro de dépôt: 95401962.6
(22) Date de dépôt: 28.08.1995
(51) Int. Cl.: C12M 1/06, C12M 3/02

(54) **Bioreacteur, en particulier pour micro-gravite**

(30) Priorité: 02.09.1994 FR 9410567
(71) Demandeur: AGENCE SPATIALE EUROPEENNE, F-75738 Paris Cedex 15 (FR)
(72) Inventeur: Mäusli, Pierre-Alain, CH-1033 Cheseaux (CH)
(74) Mandataire: Orès, Bernard

(57) **Abrégé**

L'invention concerne un bioréacteur, en particulier pour micro-gravité, comprenant au moins une chambre de culture cellulaire, un moyen pour lui fournir de l'oxygène, et un moyen d'agitation de la culture cellulaire. Selon l'invention, la chambre comporte deux éléments de boîtier (1, 2) présentant une première (1', 9) et une deuxième (2', 9') régions de paroi, ainsi qu'un premier (3) et un deuxième (4) éléments séparateurs s'étendant depuis respectivement la première (1', 9) et la deuxième (2', 9') régions de paroi en direction de respectivement la deuxième (2', 9') et la première (1', 9) régions de paroi. La ou les région(s) de séparation des éléments séparateurs présente(nt) une hauteur (h) inférieure à la distance (D) entre la première (1', 9) et la deuxième (2',9') régions de paroi, et le moyen d'agitation comporte un moyen (7) pour déplacer les deux éléments de boîtier (1, 2) l'un par rapport à l'autre.

## Description

La présente invention a pour objet un bioréacteur, en particulier pour micro-gravité, comprenant au moins une chambre de culture cellulaire, un moyen pour fournir de l'oxygène à ladite chambre, et un moyen d'agitation de la culture cellulaire permettant à celle-ci d'entrer en contact avec l'oxygène fourni à la chambre.

Dans les conditions de micro-gravité telles qu'on en rencontre dans les satellites, il est particulièrement souhaitable que l'agitation du liquide de culture cellulaire soit le plus possible réduite. D'autre part, les bioréacteurs utilisables dans des conditions de micro-gravité imposent des principes de construction différents de ceux destinés uniquement à des applications terrestres, en raison du fait que, dans des conditions de micro-gravité, il n'existe pas de phénomènes de convexion. Une autre contrainte est la nécessité d'une bonne aération et d'une bonne homogénéité du liquide de culture cellulaire, couplée avec une mise en oeuvre aussi automatique que possible, notamment pour le nettoyage.

Le Brevet des Etats-Unis US-5 002 890 délivré à l'Etat américain le 26 mars 1991 a pour objet un bioréacteur utilisable dans des conditions de micro-gravité et qui comporte une chambre verticale dans laquelle un ensemble de filtres rotatifs disposés en position centrale coopère avec des membranes flexibles qui sont disposées de manière à tourner annulairement autour de l'ensemble du filtre. Dans ce bioréacteur, le processus d'agitation et de mélange repose sur la dynamique des fluides et dépend de ce fait dans une large mesure de la vitesse de rotation. Un tel bioréacteur ne permet donc pas une vitesse de rotation réduite.

La présente invention a pour objet un bioréacteur permettant d'éviter au moins l'inconvénient précité.

Le bioréacteur selon l'invention est dans ce but caractérisé en ce que la chambre de culture cellulaire comporte un premier et un deuxième éléments de boîtier présentant respectivement une première et une deuxième régions de paroi qui se font face, ainsi qu'un premier et un deuxième éléments séparateurs comprenant au moins une première et une deuxième régions de séparation s'étendant depuis respectivement la première et la deuxième régions de paroi en direction respectivement de la deuxième et la première régions de paroi, en ce que la ou les régions de séparation d'au moins un des éléments séparateurs présente(nt) une hauteur inférieure à la distance entre la première et la deuxième régions de paroi, et en ce que le moyen d'agitation comporte un moyen pour déplacer le premier et le deuxième éléments de boîtier l'un par rapport à l'autre, ce qui permet de faire varier la distance entre les éléments séparateurs et/ou les éléments de boîtier.

Un bioréacteur tel que défini ci-dessus permet une oxygénation du liquide de culture cellulaire à un très faible niveau d'agitation et sans accélération significative des micro-organismes tout en permettant une homogénéité suffisante de la culture.

La première et la deuxième régions de séparation présentent avantageusement une hauteur inférieure à la distance entre la première et la deuxième régions de paroi. Ceci permet une oxygénation encore meilleure du liquide de culture cellulaire.

La première et la deuxième régions de paroi peuvent être planes.

La première et la deuxième régions de paroi peuvent être parallèles.

Au moins une région de séparation peut être plane.

Selon un mode de réalisation préféré, le premier et le deuxième éléments séparateurs présentent une première et une deuxième régions de séparation en spirale, qui peuvent avantageusement présenter des lois de croissance sensiblement identiques.

Le moyen pour déplacer le premier et le deuxième éléments de boîtier l'un par rapport à l'autre peut alors comporter un dispositif de mise en rotation alternative du premier et du deuxième éléments de boîtier l'un par rapport à l'autre. Le bioréacteur peut également comporter un dispositif de translation du premier et du deuxième éléments de boîtier l'un par rapport à l'autre de manière à faire varier la distance entre lesdits éléments de boîtier. Le bioréacteur peut comporter un moyen pour mettre en communication une entrée et une sortie de la chambre, ce qui permet en outre de réaliser une circulation de liquide par effet de pompage obtenu par la mise en action alternative du dispositif de translation.

Le dispositif de mise en rotation et le dispositif de translation peuvent être mis en action simultanément.

Le bioréacteur peut comporter un moyen pour mettre en butée les deux spirales l'une sur l'autre, par rotation relative du premier et du deuxième éléments de boîtier, de sorte que la chambre se présente sous la forme d'un tube continu.

Au moins une région de paroi peut être poreuse aux gaz au moins sur une partie de sa surface, et le moyen pour fournir de l'oxygène à la chambre peut alors comporter un moyen pour insuffler de l'oxygène à travers une dite région de paroi poreuse.

Selon un autre mode de réalisation, la première et la deuxième régions de paroi sont cylindriques. La première et la deuxième régions de paroi peuvent être par exemple coaxiales. Le moyen pour déplacer le premier et le deuxième éléments de boîtier l'un par rapport à l'autre peut alors comporter au moins un dispositif de translation relative de la première et de la deuxième régions de paroi cylindrique.

Au moins une région de séparation peut avantageusement comporter au moins un plateau cylindrique.

D'autres caractéristiques et avantages de l'invention apparaîtront mieux à la lecture de la description qui va suivre, donnée à titre d'exemple non limitatif, en liaison avec les dessins dans lesquels :
- les figures 1 et 2 représentent une vue schématique d'un bioréacteur selon l'invention respectivement en coupe verticale et en vue en plan ;
- la figure 3 représente une coupe verticale d'un bioréacteur selon un mode de réalisation préféré de l'invention ; la figure 4 étant une vue élargie de la partie de la figure 3 cerclée en I, et la figure 5 étant une vue élargie de la partie de la figure 3 cerclée en II ;
- la figure 6 représente une coupe horizontale vue de dessus du boîtier inférieur du bioréacteur de la figure 3 ;
- la figure 7 est une vue de dessus avec coupe partielle du boîtier supérieur du bioréacteur de la figure 3 ;
- les figures 8 et 9 représentent une vue schématique respectivement en coupe verticale et en vue en plan d'un bioréacteur selon un deuxième mode de réalisation de l'invention ;
- et les figures 10 et 11 représentent respectivement en coupe verticale et en vue de dessus un troisième mode de réalisation de l'invention.

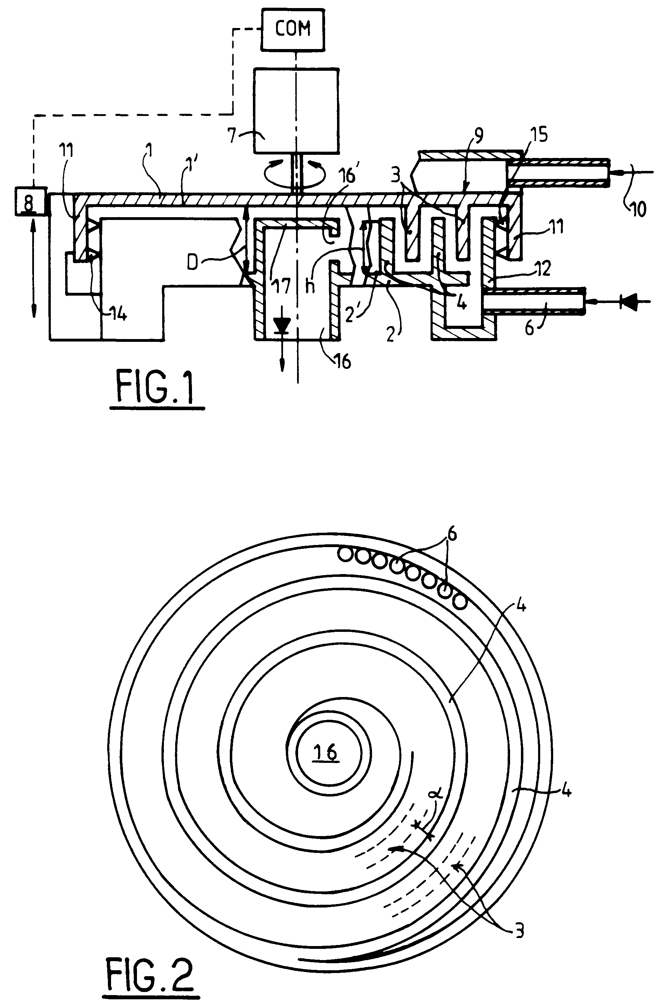

Comme représenté aux figures 1 et 2, un bioréacteur selon l'invention comporte un volume actif délimité par deux boîtiers plats complémentaires 1 et 2 dont chacun présente une région de paroi respectivement 3 et 4 s'étendant depuis une paroi extérieure respectivement 11 et 12 jusqu'à une région centrale (16, 17). L'entrée du liquide de culture cellulaire est répartie sur le pourtour de la paroi externe 12 qui présente à cet effet une pluralité de canalisations 6. La région centrale est délimitée par un bord supérieur 17 et est pourvue d'une ouverture 16, 16' de sortie du liquide. Des vannes unidirectionnelles d'entrée et de sortie, non représentées, peuvent être mises en oeuvre.

Les deux boîtiers plats 1 et 2 sont montés l'un par rapport à l'autre de manière que leurs régions de paroi (ou murs) en spirale 3 et 4 s'interpénètrent de manière à créer une surface de paroi importante qui est répartie de manière homogène dans le volume intérieur du réacteur entre les boîtiers 1 et 2, pour former une chambre de culture cellulaire. La hauteur h de l'un et/ou des deux parois en spirale 3 et 4 est telle qu'elle est inférieure à la distance nominale D₀ entre les parois internes 1' et 2' des boîtiers 1 et 2. Un moteur 7 entraîne le boîtier 1 dans une rotation alternative autour d'un axe représenté par convention comme étant vertical et un dispositif 8 permet de faire varier par translation la distance D entre les deux boîtiers 1 et 2 par rapport à la distance nominale D₀.

Le bioréacteur tel que décrit ci-dessus peut fonctionner selon trois modes :

Dans le premier mode, une fois la chambre de culture cellulaire remplie, les entrées 6 et la sortie 16 sont obturées, et le mélange est obtenu grâce au mouvement alternatif de rotation généré par le moteur 7. Ce mouvement ne change pas le volume total du réacteur mais, localement, la distance horizontale entre les parois en spirale 3 et 4 est modifiée, ce qui force un mouvement du liquide de culture cellulaire autour desdites parois. Etant donné que l'ensemble du volume est affecté de manière égale par ce mouvement, le mélange est homogène et son amplitude est réglable en agissant sur la vitesse du moteur 7 et sur l'amplitude de la rotation alternative. On peut également choisir la distance Δh entre les parois 3 et 4 et les bords intérieurs respectivement 1' et 2'.

Selon le deuxième mode de fonctionnement, une action de pompage est obtenue à partir du dispositif de commande en translation 8. Dans cette configuration les entrées 6 et la sortie 16 sont ouvertes, c'est-à-dire que les vannes unidirectionnelles précitées sont passantes. Le mélange est maintenant le résultat d'une combinaison des deux mouvements de rotation et de translation.

Le troisième mode de fonctionnement est obtenu en faisant tourner les boîtiers 1 et 2 l'un par rapport à l'autre à l'aide du moteur 7 jusqu'à ce que les murs 3 et 4 viennent en contact l'un avec l'autre. Un tube en spirale de grande longueur est ainsi obtenu et un mouvement de fluide peut être obtenu par une commande alternative du dispositif de translation 8.

Il résulte de ce qui précède que l'agitation de la suspension liquide qui constitue la culture cellulaire est obtenu par les mouvements relatifs des deux murs ou parois en spirale 3 et 4. Ces mouvements, qu'ils soient de rotation ou de translation, génèrent localement des vortex, qui sont distribués de manière homogène dans le volume du bioréacteur. L'accélération locale dépend de la vitesse relative des deux boîtiers 1 et 2 ainsi que de la distance Δh séparant le sommet d'une paroi en spirale d'un boîtier, du bord intérieur de l'autre boîtier. Il en résulte que l'accélération est ainsi réglable de manière continue à partir de la valeur 0.

Dans le premier mode décrit ci-dessus, le mélange peut conduire à un flux et à un reflux sans qu'il se produise de mélange à grande distance. On peut remédier à cet inconvénient en interconnectant les vannes de sortie et à l'entrée de manière à boucler la chambre sur elle-même. Dans le cas où un flux est généré, la distance de mélange est notamment augmentée.

L'échange de gaz peut être réalisé grâce à une région de paroi poreuse montée à la partie supérieure du boîtier 1 et à travers laquelle du gaz contenant de l'oxygène est introduit grâce à une entrée de gaz 10. La surface d'échange gazeux peut de préférence être telle qu'elle assure une distance aussi faible que possible de l'entrée de gaz de chaque point du liquide. Grâce au procédé de mélange décrit ci-dessus et à la géométrie particulière qui est décrite, le liquide est périodiquement proche des parois internes 1' et 2' des boîtiers 1 et 2, ce qui est favorable aux échanges de gaz.

Des éléments 9 sont de préférence répartis sur toute la surface supérieure du boîtier 1. Ils peuvent être réalisés en un matériau poreux étanche aux liquides ou en une membrane perméable aux gaz. On peut utiliser par exemple des céramiques, du PTFE expansé tel que celui vendu sous la Marque GORETEX, ou bien encore du silicone. Les critères de choix des emplacements sont l'accessibilité pour la maintenance, l'homogénéité de l'échange de gaz, la biocompatibilité, la résistance mécanique et la possibilité d'obtenir une stérilisation.

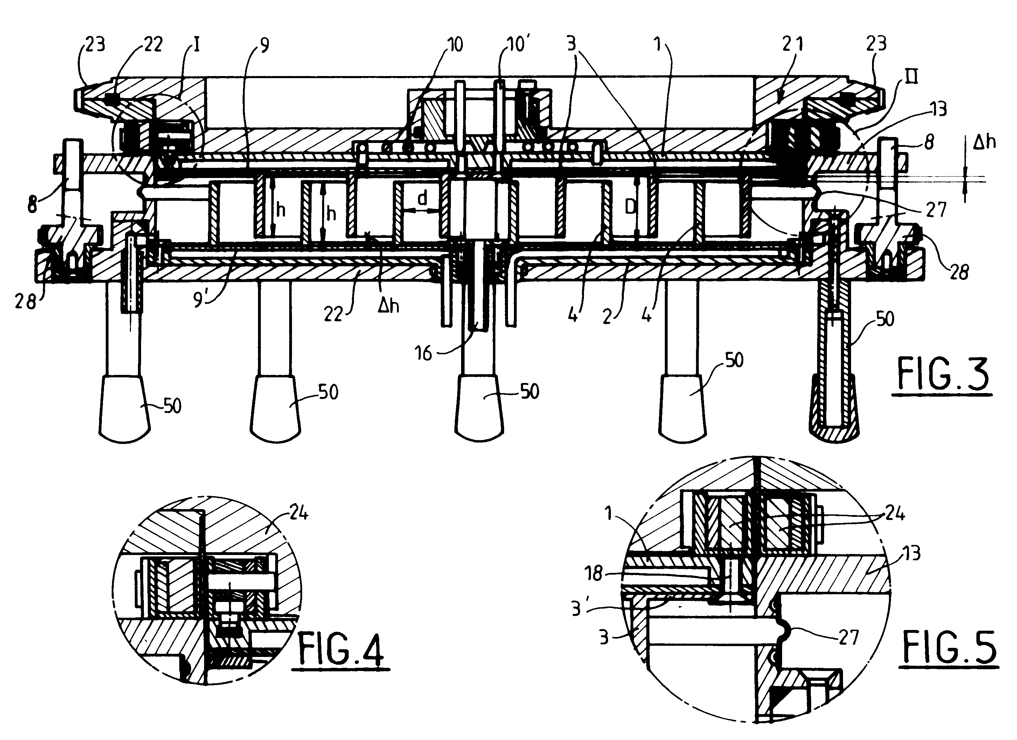

L'oxygénation et l'enlèvement du CO₂, qui doivent être réalisés en évitant toute création de bulles en micro-gravité, peuvent être obtenus comme représenté à la figure 3 à l'aide de membranes échangeuses ou de parois poreuses 9, 9'. Etant donné que la longueur de diffusion est très courte dans le liquide, ceci impose que la surface d'échange soit très grande et soit répartie de manière homogène dans le volume ou bien que la totalité du volume du liquide soit forcée de manière périodique à se trouver à proximité immédiate de la surface d'échange. Cette dernière condition est facilement réalisée avec le concept du bioréacteur décrit ci-dessus étant donné que la totalité du volume du liquide est conduite à traverser l'intervalle réglable Δh pendant le mouvement de rotation de la spirale 34.

En se reportant maintenant à la figure 3, les boîtiers 1 et 2 délimitent le volume actif de la chambre du réacteur sont placés dans un support rigide comportant un couvercle 21 et une base en deux parties 22 et 13, la partie 13 étant reliée à la partie 12 par une membrane élastique 27 qui autorise un mouvement de translation propre au réglage de l'intervalle Δh.

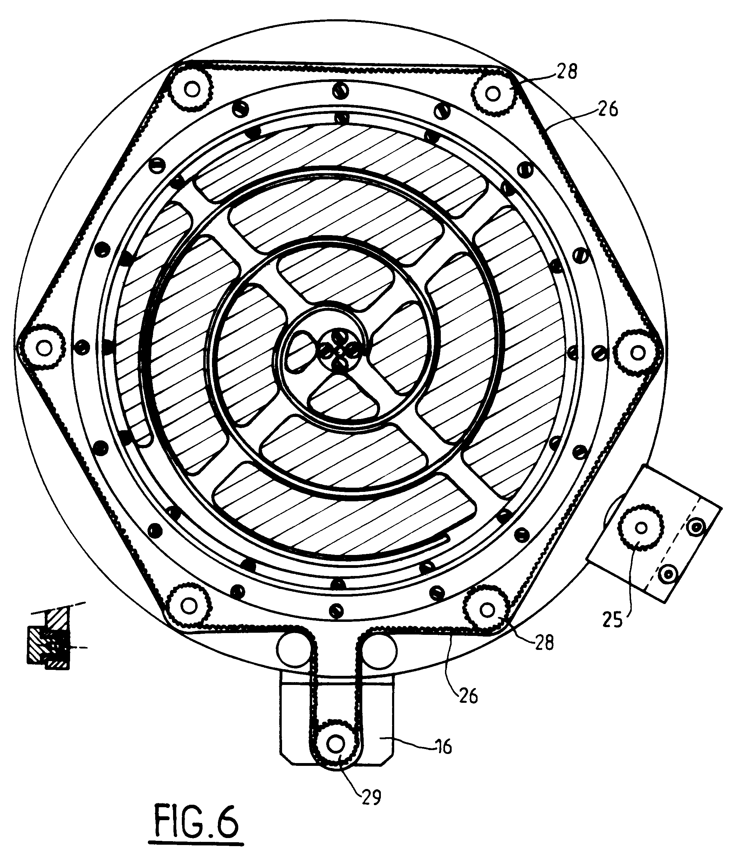

Ce mouvement de translation peut être réalisé par exemple comme représenté à la figure 6 par un dispositif d'entraînement externe 16 présentant une roue d'entraînement 29 pilotant une chaîne 26 qui fait tourner des roues dentées 28 présentant un prolongement fileté 8 s'engageant dans des parties taraudées de la pièce 13 laquelle porte le couvercle 21 et le boîtier 1 ainsi que la membrane 9.

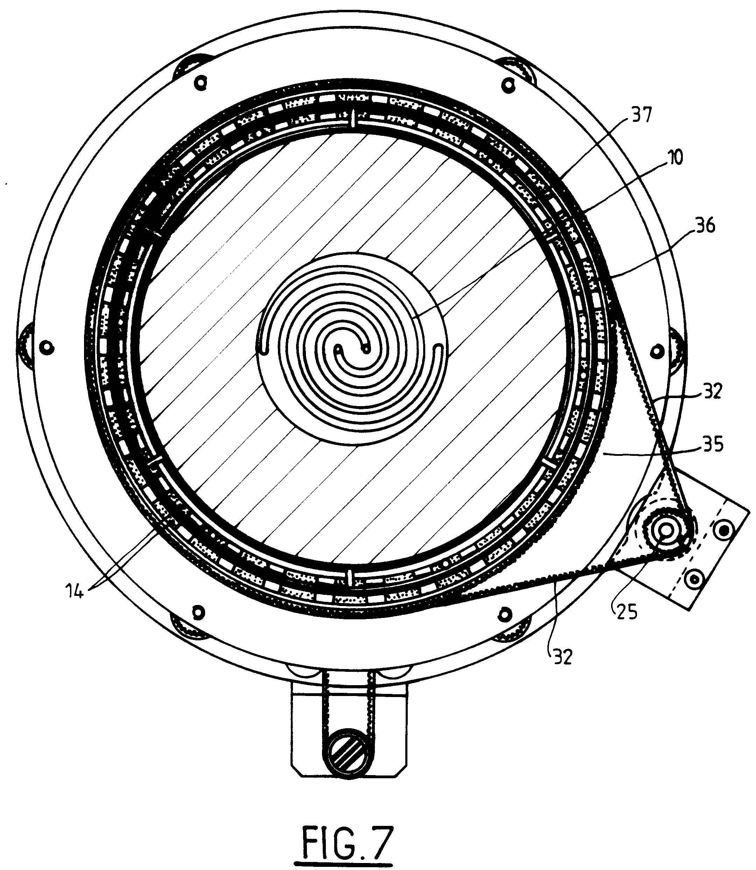

Les parties du bioréacteur qui sont mises en rotation sont les éléments 1, 3 et 9. Ils sont entraînés (voir figures 4, 5 et 7) par un dispositif magnétique à aimants 14, une première série d'aimants étant répartie sur le pourtour d'une couronne 36 entraînée en rotation par un moteur 25 à roues dentées entraînant une chaîne 32 faisant tourner une couronne dentée 35 portée par la périphérie de la couronne 36. Une deuxième série d'aimants 14 est portée par une couronne 37 interne au boîtier 1 qui est solidaire de celui-ci. A la figure 7, on voit également la circulation d'air (ou d'oxygène) qui peut être réalisée à partir de tuyaux 10 qui sont disposés en spirale, ce qui supprime la nécessité de joints rotatifs.

Les matériaux utilisés pour mettre en oeuvre le bioréacteur sont biocompatibles et étuvables de manière que chaque élément, assemblé ou non, soit stérilisable.

La structure en spirale permet un assemblage et un désassemblage faciles de même qu'un nettoyage sans grande difficulté.

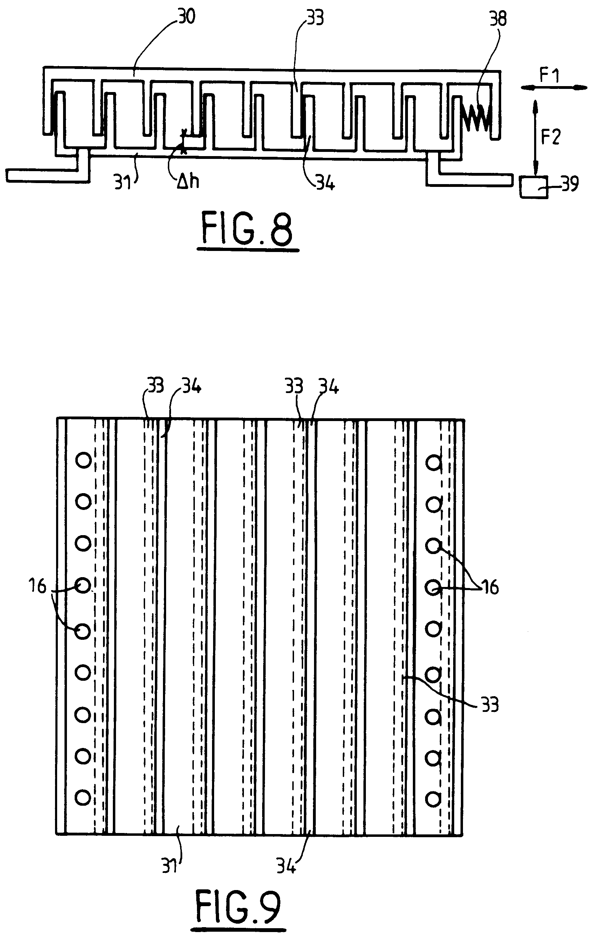

Dans les modes de réalisation présentés aux figures 8 et 9, les boîtiers supérieur et inférieur respectivement 30 et 31 sont de forme rectangulaire ou carrée et comprennent chacun une pluralité de parois longitudinales parallèles respectivement 33 et 34.

L'agitation du liquide peut être obtenue par translation dans deux directions orthogonales suivant la flèche F1 (dispositif 38) pour faire varier la distance entre les parois 33 et 34 et/ou la flèche F2 (dispositif 39) pour faire varier l'intervalle Δh. Les entrées de liquide sont référencées 6' et les sorties de liquide sont référencées 16'.

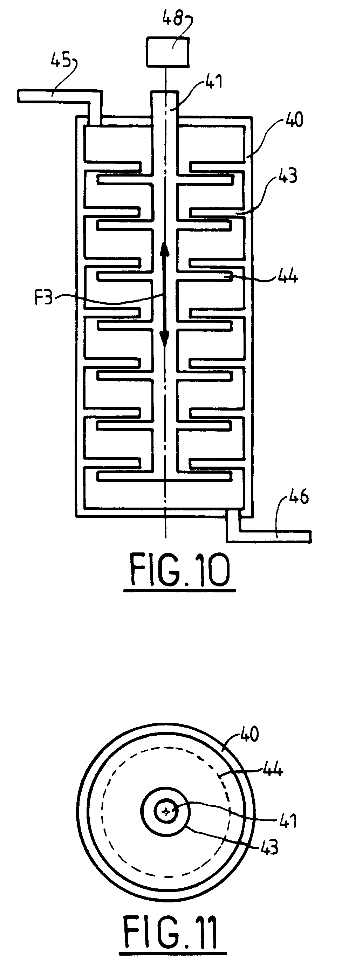

Le mode de réalisation des figures 10 et 11 met en oeuvre une structure concentrique dans laquelle le volume actif du réacteur est défini par les parties cylindriques externes 40 vues de régions de paroi 33 annulaires, imbriquées avec des régions de paroi annulaires 44 portées par un cylindre ou un piston central 41. La distance entre les parois annulaires 43 et 44 peut être modifiée, grâce au moteur 48, dans la direction de la flèche F3. L'alimentation en liquide se fait par le haut par le conduit 45 et l'évacuation par le bas, par le conduit d'évacuation 46.

## Revendications

1. Bioréacteur en particulier pour micro-gravité, comprenant au moins une chambre de culture cellulaire, un moyen pour fournir de l'oxygène à ladite chambre, et un moyen d'agitation de la culture cellulaire permettant à celle-ci d'entrer en contact avec l'oxygène fourni à ladite chambre caractérisé en ce que ladite chambre comporte un premier (1) et un deuxième (2) éléments de boîtier présentant respectivement une première (1', 9) et une deuxième (2', 9') régions de paroi qui se font face, ainsi qu'un premier (3) et un deuxième (4) éléments séparateurs comprenant au moins une première et une deuxième régions de séparation s'étendant depuis respectivement la première (1', 9) et la deuxième (2', 9') régions de paroi en direction de respectivement la deuxième (2', 9') et la première (1', 9) régions de paroi, en ce que la ou les région(s) de séparation d'au moins un des éléments séparateurs présente(nt) une hauteur (h) inférieure à la distance (D) entre la première (1', 9) et la deuxième (2',9') régions de paroi, et en ce que le moyen d'agitation comporte un moyen (7) pour déplacer le premier (1) et le deuxième (2) éléments de boîtier l'un par rapport à l'autre.

2. Bioréacteur selon la revendication 1 caractérisé en ce que la première et la deuxième régions de séparation présentent une hauteur (h) inférieure à la distance (D) entre la première (1', 9) et la deuxième (2', 9') régions de paroi.

3. Bioréacteur selon la revendication 1 ou 2 caractérisé en ce que la première (1', 9) et la deuxième (2', 9') régions de paroi sont planes.

4. Bioréacteur selon la revendication 3 caractérisé en ce que la première (1', 9) et la deuxième (2', 9') régions de paroi sont parallèles.

5. Bioréacteur selon une des revendications 1 à 4 caractérisé en ce qu'au moins une région de séparation (33, 34, 43, 44) est plane.

6. Bioréacteur selon une des revendications 1 à 4 caractérisé en ce que le premier et le deuxième éléments séparateurs présentent une première (3) et une deuxième région (4) de séparation en spirale.

7. Bioréacteur selon la revendication 6 caractérisé en ce que les spirales de la première (3) et de la deuxième (4) région de séparation en spirale présentent des lois de croissance sensiblement identiques.

8. Bioréacteur selon une des revendications 6 ou 7 caractérisé en ce que ledit moyen pour déplacer le premier (1) et le deuxième (2) élément de boîtier l'un par rapport à l'autre comporte un dispositif (7) de mise en rotation alternative du premier (1) et du deuxième (2) élément de boîtier l'un par rapport à l'autre de manière à faire varier la distance (d) entre les éléments séparateurs (3, 4).

9. Bioréacteur selon une des revendications 6 à 8 caractérisé en ce que ledit moyen pour dépacler le premier (1) et le deuxième (2) éléments de boîtier l'un par rapport à l'autre comporte un dispositif (8) de translation du premier (1) et du deuxième éléments de boîtier l'un par rapport à l'autre de manière à faire varier la distance (D) entre lesdits éléments de boîtier (1, 2).

10. Bioréacteur selon une des revendications 8 ou 9 caractérisé en ce qu'il comporte un moyen pour mettre en communication une entrée (6) et une sortie (16) de la chambre.

11. Bioréacteur selon la revendication 10 caractérisé en ce qu'il comporte un moyen de commande (COM) pour actionner simultanément le dispositif (7) de mise en rotation et le dispositif (8) de translation.

12. Bioréacteur selon une des revendications 9 à 11 caractérisé en ce qu'il comporte un moyen (7) pour mettre en butée les deux régions de séparation (3, 4) en spirale l'une sur l'autre, par rotation relative du premier (1) et du deuxième éléments (2) de boîtier, de sorte que la chambre se présente sous la forme d'un tube continu.

13. Bioréacteur selon une des revendications précédentes caractérisé en ce qu'au moins une région de paroi (9, 9') est poreuse aux gaz au moins sur une partie de sa surface et en ce que le moyen pour fournir de l'oxygène à la chambre comporte un moyen (10, 10') pour insuffler de l'oxygène à travers une dite région de paroi poreuse.

14. Bioréacteur selon une des revendications 1 ou 2 caractérisé en ce que la première et la deuxième régions de paroi sont cylindriques.

15. Bioréacteur selon la revendication 14 caractérisé en ce que la première (40) et la deuxième (41) régions de paroi sont coaxiales.

16. Bioréacteur selon la revendication 15 caractérisé en ce que ledit moyen pour déplacer le premier et le deuxième éléments de boîtier l'un par rapport à l'autre comporte au moins un dispositif (38, 39, 48) de translation relative de la première (40) et de la deuxième (41) région de paroi cylindrique.

17. Bioréacteur selon une des revendications 14 à 16 caractérisé en ce qu'au moins une région de séparation comporte au moins un plateau cylindrique (43, 44).
